# EUROPEAN PATENT APPLICATION

(11) **EP 4 108 760 A1**
(43) Date of publication of application: **28.12.2022**
(21) Application number: 21757413.6
(22) Date of filing: 17.02.2021
(51) Int. Cl.: C12M 1/32, C12M 3/00, A61M 37/00

(54) **METHOD FOR PRETREATMENT OF CELL TRANSPLANTATION, DEVICE FOR PRETREATMENT OF CELL TRANSPLANTATION, AND UNIT FOR PRETREATMENT OF CELL TRANSPLANTATION**

(30) Priority: 19.02.2020 JP 2020026243
(71) Applicant: TOPPAN INC., Tokyo 110-0016 (JP)
(72) Inventor: KODAMA Yoshihiro, Tokyo 110-0016 (JP)
(74) Representative: TBK
(86) International application number: PCT/JP2021/006002
(87) International publication number: WO 2021/166977

(57) **Abstract**

A cell transplantation pretreatment method, a cell transplantation pretreatment device, and a cell transplantation pretreatment unit are provided, which are capable of improving the efficiency of loading grafts into a transplantation device. The cell transplantation pretreatment method includes the steps of: transporting a cell group cultured in a culture recess to a pretreatment recess 21 using a culture tray including a plurality of the culture recesses arranged according to a first arrangement, a pretreatment tray 20 including a plurality of the pretreatment recesses 21 arranged according to a second arrangement, and a transplantation device 30 including a needle shaped portion 31 having a tubular shape configured to allow entry and exit of a graft containing the cell group, the transplantation device including a plurality of the needle shaped portions 31 arranged according to the second arrangement; and loading the cell groups simultaneously from the plurality of pretreatment recesses 21 into the plurality of needle shaped portions 31.

## Description

### [Technical Field]

The present disclosure relates to a cell transplantation pretreatment method, a cell transplantation pretreatment device, and a cell transplantation pretreatment unit.

### [Background Art]

Techniques have been developed for transplanting a graft which is obtained by culturing cells collected from a living body into an intradermal or subcutaneous site in a living body. For example, regeneration of hair has been performed by purifying groups of cells that contribute to formation of hair follicles, which are organs that produce hair, and transplanting the groups of cells into intradermal sites.

Hair follicles are formed by interactions between epithelial cells and mesenchymal cells. For good hair regeneration, it is desirable that the transplanted cell groups generate hair follicles having a normal tissue structure and capable of a normal hair growth cycle. Therefore, various research and development have been made to provide methods for production and transplantation of cell groups that can produce such hair follicles (for example, see PTLs 1 to 3). Further, cell transplantation by surgery imposes a heavy burden on the body and a lot of time and effort are required for the transplantation. Accordingly, there is a great need for transplantation methods that improve the safety and simplicity thereof, and various cell transplantation methods have been developed to meet such needs (for example, see PTL 4).

### [Citation List]

### [Patent Literature]

PTL 1: WO2017/073625
PTL 2: WO2012/108069
PTL 3: JP 2008-29331 A
PTL 4: JP 2019-064653 T

### [Summary of the Invention]

### [Technical Problem]

In an example of the transplantation methods that are being developed as described above, a graft containing a cell group that contributes to regeneration of hair follicles is loaded into a needle shaped portion disposed in a transplantation device, and injected into a living body for transplantation. The needle shaped portion is disposed in the transplantation device at a position corresponding to a transplantation target position of the cell group. Accordingly, the transplantation device can transplant the cell group to the transplantation target position.

For hair regeneration, the positions and intervals of the cell groups to be transplanted into the skin are important. From the viewpoint of hair regeneration, the transplantation target positions of the cell groups may be set to an irregular arrangement or a regular arrangement according to the application of the transplant site. On the other hand, in many culture trays configured to be suitable for culturing the cell groups, the culture recesses are arranged in a regular matrix according to the application, such that the culture recesses for culturing the cell groups are used row by row in sequence. Therefore, the positions of the needle shaped portions and the positions of the culture recesses are not aligned with each other, which makes it difficult to load the grafts simultaneously from a plurality of culture recesses into the needle shaped portions, and loading of the grafts into the needle shaped portions is a process that requires time and effort.

The present disclosure has been made to provide a cell transplantation pretreatment method, a cell transplantation pretreatment device, and a cell transplantation pretreatment unit capable of improving the efficiency of loading grafts into a transplantation device.

### [Solution to Problem]

A cell transplantation pretreatment method for solving the above problem includes the steps of: transporting a cell group cultured in a culture recess to a pretreatment recess using a culture tray including a plurality of the culture recesses arranged according to a first arrangement, a pretreatment tray including a plurality of the pretreatment recesses arranged according to a second arrangement, and a transplantation device including a needle shaped portion having a tubular shape configured to allow entry and exit of a graft containing the cell group, the transplantation device including a plurality of the needle shaped portions arranged according to the second arrangement; and loading the cell groups simultaneously from the plurality of pretreatment recesses into the plurality of needle shaped portions.

A cell transplantation pretreatment device for solving the above problem includes: a transport unit that transports a cell group cultured in a culture recess to a pretreatment recess using a culture tray including a plurality of the culture recesses arranged according to a first arrangement and a pretreatment tray including a plurality of the pretreatment recesses arranged according to a second arrangement; and a loading unit that loads the cell groups simultaneously from the plurality of pretreatment recesses into a plurality of needle shaped portions using a transplantation device and the pretreatment tray, the transplantation device including the plurality of needle shaped portions having a tubular shape configured to allow entry and exit of a graft containing the cell group, the plurality of needle shaped portions being arranged according to the second arrangement.

A cell transplantation pretreatment unit for solving the above problem includes: a culture tray including a plurality of culture recesses arranged according to a first arrangement; a pretreatment tray including a plurality of pretreatment recesses arranged according to a second arrangement, the pretreatment tray being configured such that cell groups cultured in the plurality of culture recesses are transported to the plurality of pretreatment recesses; and a transplantation device including a plurality of needle shaped portions having a tubular shape configured to allow entry and exit of a graft containing the cell group, the plurality of needle shaped portions being arranged according to the second arrangement, and the transplantation device being configured such that the cell groups are able to be loaded simultaneously from the plurality of pretreatment recesses into the plurality of needle shaped portions.

Since the grafts can be simultaneously loaded into the plurality of needle shaped portions of the transplantation device, cells can be simultaneously transplanted to many transplantation target positions. This greatly improves the efficiency from the time after culturing cells to transplantation of cells. While the plurality of needle shaped portions of the transplantation device are set to an irregular arrangement or a regular arrangement according to the application of the transplant site, the plurality of culture recesses of the culture tray are arranged irrespectively of the arrangement of the needle shaped portions. Such a difference in the arrangement between the culture recesses and the needle shaped portions causes difficulty in simultaneous loading of the grafts into the plurality of needle shaped portions, and requires great time and effort since the needle shaped portions are collectively moved relative to each of the culture recesses to load the grafts one by one into the needle shaped portions. In this regard, according to the above configuration, the pretreatment tray includes the pretreatment recesses arranged according to the arrangement of the needle shaped portions of the transplantation device. Accordingly, the needle shaped portions can be easily inserted into the pretreatment recesses, reducing the time and effort required for loading the grafts from the pretreatment tray into the transplantation device. This improves the efficiency of loading the grafts into the transplantation device.

In the above cell transplantation pretreatment method, the pretreatment recess may have an inner diameter smaller than that of the culture recess.

In aspiration of the grafts from the pretreatment recesses into the needle shaped portions, a cell group contained in the graft is less likely to be aspirated compared with a liquid such as a protective liquid contained in the graft. In this regard, according to the above method, since the inner diameter of the pretreatment recess is smaller than the inner diameter of the culture recess, a gap formed between the pretreatment recess and the needle shaped portion can be reduced when the graft is aspirated from the pretreatment recess into the needle shaped portion. As a result, it is possible to increase a force for aspirating the graft from the pretreatment recess into the needle shaped portion, and thus improve the accuracy and efficiency in aspiration of the cell group contained in the graft from the pretreatment recess into the needle shaped portion.

In the above cell transplantation pretreatment method, an engaging portion may be provided to block insertion of the needle shaped portion while forming a gap between a tip of the needle shaped portion inserted in the pretreatment recess and a bottom of the pretreatment recess, and the step of loading the cell groups into the needle shaped portions may be performed in a state in which the engaging portion blocks insertion of the needle shaped portion.

According to the above method, in loading of the grafts into the transplantation device, the tip of the needle shaped portion is prevented from coming into contact with the bottom of the pretreatment recess. Accordingly, the cell groups can be loaded into the transplantation device while maintaining the state of the needle shaped portions suitable for transplantation.

In the above cell transplantation pretreatment method, the pretreatment recess may have a bottom shape different from that of the culture recess.

According to the above method, the bottom of the culture recess can be suitably shaped for culturing cells, and the bottom of the pretreatment recess can be suitably shaped for loading the graft into the transplantation device. For example, the bottom of the culture recess can be a flat surface so that the cell group can easily adhere to the bottom, and the bottom of the pretreatment recess can be a hemispherical recess so that the cell group can be easily aspirated. This improves the efficiency of cell culture and loading operation.

In the above cell transplantation pretreatment method, the first arrangement and the second arrangement may satisfy at least one of the following conditions 1 to 3: (Condition 1) an arrangement direction of the culture recesses arranged according to the first arrangement and an arrangement direction of the pretreatment recesses arranged according to the second arrangement are different from each other; (Condition 2) an interval between the culture recesses adjacent to each other and an interval between the pretreatment recesses adjacent to each other are different from each other; and (Condition 3) a number of culture recesses in the arrangement direction of the culture recesses arranged according to the first arrangement and a number of pretreatment recesses in the arrangement direction of the pretreatment recesses arranged according to the second arrangement are different from each other.

In a configuration in which a difference between the first arrangement and the second arrangement satisfies at least one of the above conditions 1 to 3, loading the grafts from the culture recesses into the needle shaped portions requires the needle shaped portions arranged according to the second arrangement to be collectively moved relative to each of the culture recesses to load the grafts one by one into the needle shaped portions. In this regard, according to the method in which the pretreatment recesses and the needle shaped portions are arranged according to a common second arrangement, the effects described above are more reliably achieved.

In the above cell transplantation pretreatment method, the culture recess may have a depth different from that of the pretreatment recess.

According to the above method, in which the depth of the culture recess and the depth of the pretreatment recess can be individually set, the depth of the culture recess can be suitably sized for culture and the depth of the pretreatment recess can be suitably sized for loading the graft into the needle shaped portion. Further, when the depth of the pretreatment recess is greater than the depth of the culture recess, the grafts can be transported from a plurality of culture recesses to a single pretreatment recess to increase the quantity of grafts to be loaded into the needle shaped portion. When the depth of the pretreatment recess is smaller than the depth of the culture recess, the quantity of grafts can be adjusted to the quantity that can be loaded into the needle shaped portion.

In the above cell transplantation pretreatment method, the pretreatment recess may have a shape in which an outer peripheral surface of the needle shaped portion is able to fit, and the step of loading the cell groups into the needle shaped portions may be performed in a state in which the outer peripheral surface of the needle shaped portion is fitted in the pretreatment recess.

According to the above method, it is possible to prevent the positions of the needle shaped portions from being displaced from the pretreatment recesses during loading of the grafts into the needle shaped portions. This prevents a failure in loading of the graft into the needle shaped portion due to displacement of the position of the needle shaped portion from the pretreatment recess.

In the above cell transplantation pretreatment method, the pretreatment tray and the transplantation device may include a structure for positioning the transplantation device relative to the pretreatment tray in a direction perpendicular to a depth direction of the pretreatment recess.

According to the above method, positioning of the transplantation device relative to the pretreatment tray can be accurately performed. In particular, this is convenient when positioning is performed manually.

In the above cell transplantation pretreatment method, the pretreatment tray may be transparent.

According to the above configuration, it is possible to transport the cell groups to the pretreatment recesses and load the cell groups into the needle shaped portions while observing the inside of the pretreatment recesses. Accordingly, these steps can be smoothly performed. Further, it is also possible to check whether there is any residue in the pretreatment recess after the cell groups are loaded into the needle shaped portions.

### [Brief Description of the Drawings]

Fig. 1 is a configuration diagram illustrating a device configuration of an embodiment of a cell transplantation pretreatment device.
Fig. 2 is a configuration diagram illustrating a device configuration of an embodiment of a cell transplantation pretreatment device.
Fig. 3 is a cross-sectional view illustrating a state before a graft is loaded into a needle shaped portion from a pretreatment tray.
Fig. 4 is a plan view illustrating a modified example of a transplantation pretreatment unit.
Fig. 5 is a plan view illustrating another modified example of a transplantation pretreatment unit.
Fig. 6 is a plan view illustrating still another modified example of a transplantation pretreatment unit.
Fig. 7 is a cross-sectional view illustrating a modified example of a culture tray.
Fig. 8 is a cross-sectional view illustrating a modified example of a pretreatment tray.
Fig. 9 is a cross-sectional view illustrating another modified example of a pretreatment tray.
Fig. 10 is a cross-sectional view illustrating still another modified example of a pretreatment tray.
Fig. 11 is a cross-sectional view illustrating still another modified example of a pretreatment tray.
Fig. 12 is a perspective view illustrating still another modified example of a pretreatment tray.
Fig. 13 is a perspective view illustrating still another modified example of a pretreatment tray.

### [Description of the Embodiments]

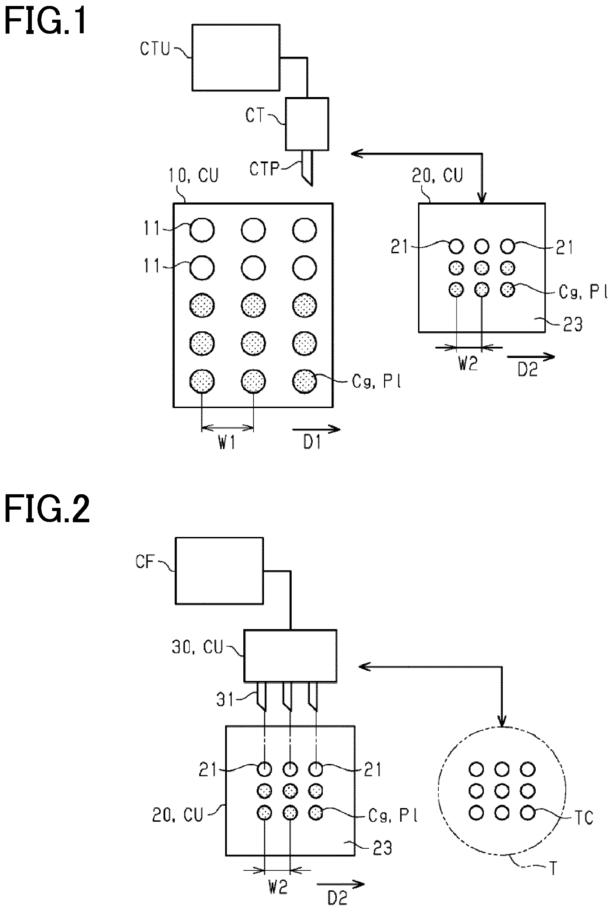

With reference to Figs. 1 and 2, an embodiment of a cell transplantation pretreatment unit, a cell transplantation pretreatment device, and a cell transplantation pretreatment method will be described.

### [Grafts]

A cell transplantation unit is used for transplanting grafts. A graft includes a cell group Cg to be transplanted into a living body and a protective liquid Pl for protecting the cell group Cg. A target site TC into which a graft is transplanted may be, for example, at least one of an intradermal layer and a subcutaneous layer, or tissues in organs or the like.

The cell group Cg may be an aggregate of a plurality of aggregated cells or an aggregate of a plurality of cells joined by intercellular junctions, or may be composed of a plurality of dispersed cells. Further, cells constituting the cell group Cg may be undifferentiated cells or cells that have been differentiated. The cell group Cg may include both undifferentiated cells and differentiated cells. Examples of the cell group Cg include masses of cells which are spheroids, germs, primordia, tissues, organs, organoids, bud organoids, mini organs, and the like.

The cell group Cg has an ability to control tissue formation in a living body when positioned in the target site TC. An example of the cell group Cg is a cell condensate containing stem cells. For example, the cell group Cg may be positioned in the intradermal or subcutaneous site to contribute to hair growth or hair restoration. The cell group Cg has an ability to function as a hair follicle organ, an ability to differentiate into a hair follicle organ, an ability to induce or promote formation of a hair follicle organ, an ability to induce or promote formation of hair in a hair follicle, or the like. The cell group Cg may include cells that contribute to control of hair color, such as pigment cells or stem cells that differentiate into pigment cells, or may include vascular cells.

The cell group Cg in the present embodiment is a primitive organ germ. The organ germs include mesenchymal cells and epithelial cells. Examples of the organ germs include hair follicle germs that differentiate into hair follicle organs, liver germs, kidney germs, pancreatic germs, neural germ cells and vascular germ cells. The hair follicle germs are prepared by, for example, culturing a mixture of mesenchymal cells derived from mesenchymal tissues such as dermal papilla and epithelial cells derived from epithelial tissues located in a bulge region or a hair bulb base under predetermined conditions. It should be noted that the process of preparing hair follicle germs is not limited to the above example. In addition, the mesenchymal cells and epithelial cells used for preparation of hair follicle germs may also be derived from any tissue. These cells may be derived from a hair follicle organ, may be derived from an organ different from the hair follicle organ, or may be derived from a pluripotent stem cell.

The cell group Cg may not be necessarily a cell group that contributes to hair growth or hair restoration, and may be a cell group Cg that exhibits desired effects when positioned in at least one of the intradermal and subcutaneous sites or in a tissue of an organ or the like. For example, a cell group to be transplanted into a skin region may be a cell group Cg that exhibits effects in cosmetic use such as elimination of wrinkles or improvement in moisturizing state in the skin.

The protective liquid Pl may be any liquid that is unlikely to hinder the viability of cells, and is preferably a liquid that has a minimal influence on a living body when injected into the living body. For example, the protective liquid Pl may be physiological saline, liquid that protects the skin such as petrolatum or lotion, or a mixture of these liquids. The protective liquid Pl may be a culture medium for culturing cells or a liquid exchanged from the culture medium. The protective liquid Pl may contain additive components such as a nutrient composition. Further, a liquid material containing the cell group Cg and the protective liquid Pl may be a low viscosity fluid or a high viscosity fluid.

### [Cell Transplantation Pretreatment Unit]

Fig. 1 is a process diagram showing a transport step constituting the cell transplantation pretreatment method, and Fig. 2 is a process diagram showing a pretreatment step constituting the cell transplantation pretreatment method. As shown in Fig. 1, the cell transplantation pretreatment device uses a cell transplantation pretreatment unit CU. The cell transplantation pretreatment device includes a transport unit CT. The cell transplantation pretreatment unit CU includes a culture tray 10 and a pretreatment tray 20. Further, as shown in Fig. 2, the cell transplantation pretreatment unit CU includes a transplantation device 30. The cell transplantation pretreatment device includes a loading unit CF.

The culture tray 10 is a tray for culturing the cell group Cg to be transplanted into a living body. The pretreatment tray 20 is a tray used when the graft is transported from the culture tray 10 and loaded into the transplantation device 30. The transplantation device 30 is a device for transplanting a graft into a living body from the pretreatment tray 20.

The transport unit CT transports the graft from the culture tray 10 to the pretreatment tray 20. The transport unit CT includes an aspiration unit CTP, such as a single pipette or a dropper, and a controller CTU that controls the operation of the aspiration unit CTP. The aspiration unit CTP aspirates the graft from a culture recess 11 of the culture tray 10 and dispenses it into a pretreatment recess 21 of the pretreatment tray 20. For example, the controller CTU stores the positions of the culture recesses 11 of the culture tray 10 and the positions of the pretreatment recesses 21 of the pretreatment tray 20, determines the destination of the aspiration unit CTP, and causes the aspiration unit CTP to aspirate and dispense the graft.

The process performed by the transport unit CT, that is, transporting the graft from the culture tray 10 to the pretreatment tray 20 may also be performed by the user of the cell transplantation pretreatment device. In other words, the transport unit CT injects the cell group Cg into the pretreatment recess 21 after it moves from the culture recess 11 to the pretreatment recess 21 while holding the cell group Cg in a dropper or a pipette. The transport unit CT may be a robot that automates a series of these operations. Further, the transport unit CT may be a group of robots performing respective steps to automate a series of operations. In addition, part or all of the process performed by the transport unit CT may not be automated.

As shown in Fig. 2, the loading unit CF loads the graft from the pretreatment tray 20 into the transplantation device 30. The loading unit CF aspirates the graft from the pretreatment recess 21 of the pretreatment tray 20 into a needle shaped portion 31 of the transplantation device 30 to thereby load the graft into the transplantation device 30. The loading unit CF performs this operation with a plurality of pretreatment recesses of the pretreatment tray 20 and a plurality of needle shaped portions 31 of the transplantation device 30 so that grafts are simultaneously loaded from the plurality of pretreatment recesses 21 into the plurality of needle shaped portions 31. The loading unit CF may be, for example, an aspiration device such as an aspiration pump.

The process performed by the transport unit CT, that is, aligning the positions of the pretreatment tray 20 with the positions of the transplantation device 30 and loading the grafts from the pretreatment tray 20 into the transplantation device may be performed by the user of the cell transplantation pretreatment device. In other words, the loading unit CF loads the grafts from the pretreatment recess 21 into the needle shaped portion 31 after it aligns the positions of the respective pretreatment recesses 21 with the positions of the respective needle shaped portions 31. The loading unit CF may be a robot that automates a series of these operations. Further, the loading unit CF may be a group of robots performing respective steps to automate a series of operations. In addition, part or all of the process performed by the loading unit CF may not be automated.

### [Culture Tray]

Referring back to Fig. 1, the culture tray 10 includes a plurality of culture recesses 11, which are recesses for culturing cells collected from a collection target. In the culture recesses 11, the cell groups Cg are cultured until they become a state suitable for transplantation. The material of the culture tray 10 and the shape of the culture recesses 11 are appropriately selected depending on the characteristics of the cell groups Cg to be cultured. The culture recesses 11 are arranged according to a first arrangement in the culture tray 10. The first arrangement may be an arrangement of all the culture recesses 11 or an arrangement of the plurality of culture recesses 11 repeated in a predetermined direction among all the culture recesses 11. When the first arrangement is an arrangement of all the culture recesses 11, the culture recesses 11 may be arranged irregularly.

For example, in the first arrangement, three culture recesses 11 are arranged in the first arrangement direction D1. The culture recesses 11 adjacent to each other are arranged with a culture interval W1. The three culture recesses 11 arranged in the first arrangement direction D1 are one row of the culture recesses 11. In the first arrangement, five rows of the culture recesses 11 are arranged in a matrix form in the first arrangement direction D1 and a direction perpendicular to the first arrangement direction D1.

The inner diameter of the culture recess 11 is a size sufficient to culture the cell group Cg. The inner diameter of the culture recess 11 is preferably large so that a drug, a medium, or the like can be easily inserted and removed from the culture recess 11.

Further, the culture interval W1 may be different among the culture recesses 11 in one row or in the first arrangement. Further, the inner diameter of each culture recess 11 may be different from the inner diameter of other culture recesses 11.

### [Pretreatment Tray]

The pretreatment tray 20 includes a plurality of pretreatment recesses 21 which are recesses for receiving the cell groups Cg cultured in the culture tray 10. The cell groups Cg transported from the culture recesses 11 are received in the pretreatment recesses 21, and then loaded into the transplantation unit from the pretreatment recesses 21. The shape of the pretreatment recesses 21 of the pretreatment tray 20 and the like are appropriately selected according to the loading of the cell groups Cg.

The pretreatment recess 21 is capable of holding the cell group Cg together with the protective liquid Pl for a predetermined period of time. The pretreatment tray 20 provided with the pretreatment recesses 21 can prevent the protective liquid Pl from evaporating or prevent the protective liquid Pl from wetting and spreading on a surface of the pretreatment tray 20 compared with the case where the pretreatment tray 20 has no pretreatment recess 21, for example, the pretreatment tray 20 having a plate shape. This prevents the cell groups Cg from drying or prevents a change in component ratio of the protective liquid PI, and thus improves the accuracy in engraftment of the cell groups Cg.

According to the pretreatment recesses 21, the protection conditions of the protective liquid Pl can be changed to other conditions in the pretreatment tray 20. Examples of the change in protection conditions of the protective liquid Pl include introducing another protective liquid Pl into the pretreatment recesses 21 after the cell groups Cg are transported to the pretreatment recesses 21, or transporting the cell groups Cg to the pretreatment recesses 21 filled with another protective liquid Pl. Such changes in protection conditions of the protective liquid Pl allow for efficient changes, such as replacement and addition, of the protective liquid Pl compared with the case where the protection conditions of the protective liquid Pl are changed without transporting the cell groups Cg. Thus, changes can be easily made to obtain protection conditions with high safety or protection conditions with a long shelf life. Further, such changes in protection conditions of the protective liquid Pl can prevent occurrence of errors in the inspection results due to color change, turbidity, presence of impurities in the protective liquid Pl, or the like which may be caused by long-term cell culture. Moreover, when a change to the protection conditions with high safety is made immediately before the cell transplantation, it is possible to relax the requirements for the safety of the protective liquid Pl at the time of cell culture and improve the safety of the protective liquid Pl at the time of cell transplantation.

The pretreatment recesses 21 are arranged according to a second arrangement in the pretreatment tray 20. The second arrangement may be an arrangement of all the pretreatment recesses 21 or an arrangement of the plurality of pretreatment recesses 21 repeated in a predetermined direction among all the pretreatment recesses 21. When the second arrangement is an arrangement of all the pretreatment recesses 21, the pretreatment recesses 21 may be arranged irregularly.

For example, in the second arrangement, three pretreatment recesses 21 are arranged in the second arrangement direction D2. The pretreatment recesses 21 adjacent to each other are arranged with a pretreatment interval W2. The three pretreatment recesses 21 arranged in the second arrangement direction D2 are one row of the pretreatment recesses 21. In all the pretreatment recesses 21, three rows of the pretreatment recesses 21 are arranged in a matrix form in the second arrangement direction D2 and a direction perpendicular to the second arrangement direction D2.

The first arrangement and the second arrangement satisfy at least one of the following conditions 1 to 3:
(Condition 1) The first arrangement direction D1 in which the culture recesses 11 are arranged according to the first arrangement and the second arrangement direction D2 in which the pretreatment recesses 21 are arranged according to the second arrangement are different from each other.

(Condition 2) The culture interval W1 between the adjacent culture recesses 11 and the pretreatment interval W2 between the adjacent pretreatment recesses 21 are different from each other.

(Condition 3) The number of culture recesses 11 in the first arrangement direction D1 in which the culture recesses 11 are arranged according to the first arrangement and the number of pretreatment recesses 21 in the second arrangement direction D2 in which the pretreatment recesses 21 are arranged according to the second arrangement are different from each other.

In the example shown in Fig. 1, the conditions 1 and 3 are satisfied among the conditions 1 to 3. The pretreatment recesses 21 for one row in the pretreatment tray 20 correspond to the needle shaped portions 31 for one row in the transplantation device 30. The needle shaped portions 31 may be simultaneously inserted into all or part of the pretreatment recesses 21 provided in the transplantation device 30.

That is, a plurality of needle shaped portions 31 may be simultaneously inserted into a plurality of pretreatment recesses 21. Further, the pretreatment recesses 21 arranged according to the first arrangement may be configured so that all the needle shaped portions 31 arranged according to the second arrangement can be inserted, or may be designed so that the needle shaped portions 31 of a plurality of transplantation devices 30 can be simultaneously inserted.

The pretreatment tray 20 includes an outer peripheral portion in which no pretreatment recess 21 is disposed. The pretreatment recesses 21 may be located in the center part of the pretreatment tray 20 compared with the culture recesses 11. The culture conditions such as illuminance, temperature and the amount of carbon dioxide tend to vary in the outer peripheral portion of the pretreatment tray 20 compared with those in the center part of the pretreatment tray 20. According to the configuration in which the pretreatment recesses 21 arranged according to the second arrangement are located in the center part of the pretreatment tray 20, it is possible to prevent the state of the cell groups Cg from varying. Further, in the pretreatment tray 20 in which the pretreatment recesses 21 are located in the center part, the outer peripheral portion of the pretreatment tray 20 can be provided with a grip portion 23 suitable for handling. This makes it easy to move the pretreatment tray 20.

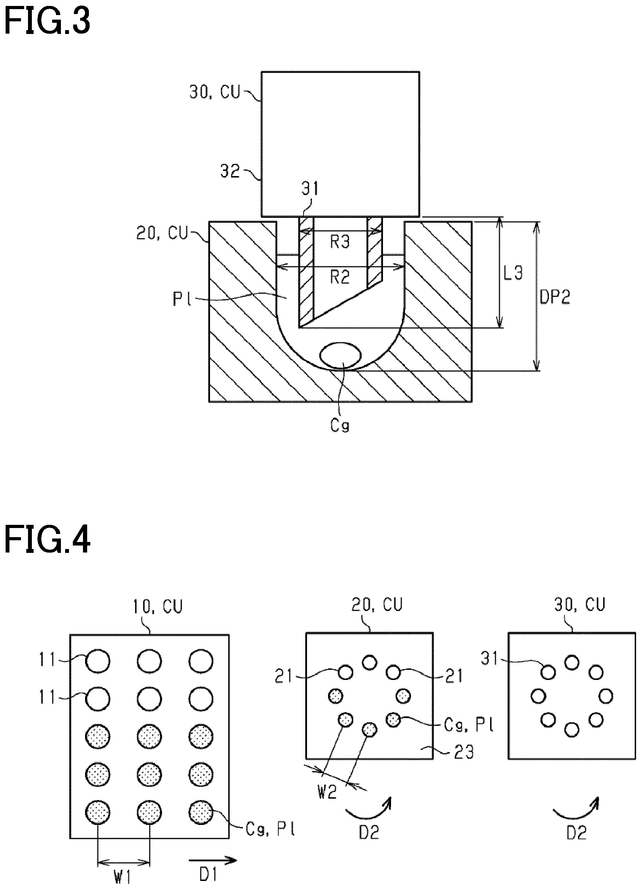

An inner diameter R2 (see Fig. 3) of the pretreatment recess 21 may be a size sufficient to accommodate the cultured cell group Cg. When the inner diameter R2 of the pretreatment recess 21 is larger than the inner diameter of the culture recess 11, the aspiration unit CTP or the needle shaped portion 31 can be easily inserted into the pretreatment recess 21, which facilitates injection and aspiration of the graft into and from the pretreatment recess 21. When the inner diameter R2 of the pretreatment recess 21 is smaller than the inner diameter of the culture recess 11, a gap formed between the pretreatment recess 21 and the needle shaped portion 31 can be reduced. Accordingly, it is possible to increase a force for aspirating the graft from the pretreatment recess 21 into the needle shaped portion 31, and thus improve the accuracy and efficiency in aspiration of the cell group Cg from the pretreatment recess 21 into the needle shaped portion 31. In addition, when the inner diameter R2 of the pretreatment recess 21 is larger than the inner diameter of the culture recess 11, the pretreatment recess 21 can be sized according to the number of cell groups Cg required for transplantation. For example, when the inner diameter of the pretreatment recess 21 is set so that a single cell group Cg can be easily aspirated by each aspiration, it is possible to prevent the cell group Cg from being left in the pretreatment recess 21 and facilitate aspiration of the cell group Cg from the pretreatment recess 21. Further, an inspection or the like that cannot be easily performed in the culture recesses 11 can be smoothly performed by changing the positions of the grafts to the pretreatment recesses 21 suitable for the inspection step. For example, when the inspection requires observation of the graft at high magnification, it is necessary to observe the graft under the condition of a shorter focal distance. In this case, although the culture recess 11 having a small inner diameter is not suitable for this observation, the observation can be performed in the pretreatment recess 21.

The pretreatment interval W2 in the pretreatment tray 20 may be constant, or two or more pretreatment intervals W2 may be provided in the pretreatment tray 20. As long as the above conditions 1 and 3 are satisfied, the culture interval W1 and the pretreatment interval W2 may be equal to each other, and the culture interval W1 and the pretreatment interval W2 may be different from each other. When the culture interval W1 and the pretreatment interval W2 are equal to each other, it is possible to transport the grafts from two adjacent culture recesses 11 to two adjacent pretreatment recesses 21, reducing the time and effort required for transporting the cell groups Cg from the culture recesses 11 to the pretreatment recesses 21.

As shown in Fig. 3, a depth DP2 of the pretreatment recess 21 may be greater or smaller than a length L3 of the needle shaped portion 31, but is preferably such that the tip of the needle shaped portion 31 does not reach the bottom of the pretreatment recess 21. The quantity of grafts that can be accommodated in the pretreatment recess 21 may be greater or less than the quantity of grafts that can be loaded into the needle shaped portion 31. When the quantity of grafts that can be accommodated in the pretreatment recess 21 is greater than the quantity of grafts that can be loaded into the needle shaped portion 31, loading from the pretreatment recess 21 into the needle shaped portion 31 can be performed multiple times. On the other hand, when the quantity of grafts that can be accommodated in the pretreatment recess 21 is smaller than the quantity of grafts that can be loaded into the needle shaped portion 31, the quantity of grafts can be optimized according to the type of the cell group Cg and the conditions for cell transplantation.

### [Transplantation Device]

The needle shaped portion 31 of the transplantation device 30 has a tubular shape extending from the proximal end to the distal end. The needle shaped portion 31 has an opening formed by, for example, truncating the tubular shape obliquely relative to the axial direction. The opening of the needle shaped portion 31 has a diameter capable of dispensing the cell group Cg, and is configured to allow entry and exit of the cell group Cg. A tip opening of the needle shaped portion 31 may have a sharp shape so that it can enter the transplant site such as a living body. The needle shaped portion 31 aspirates the graft containing the cell group Cg from the pretreatment recess 21. The needle shaped portion 31 holds the cell group Cg inside the needle shaped portion 31, and dispenses the cell group Cg in a state in which the needle shaped portion 31 is punctured into a living body T.

The needle shaped portions 31 are arranged according to the second arrangement in the transplantation device 30. That is, in the transplantation device 30, three needle shaped portions 31 are arranged in the second arrangement direction D2 with the pretreatment interval W2 as with the pretreatment recesses 21. The three needle shaped portions 31 arranged in the second arrangement direction D2 are one row of the needle shaped portions 31. In the second arrangement, three rows of the needle shaped portions 31 are arranged in a matrix form in the second arrangement direction D2 and a direction perpendicular to the second arrangement direction D2.

The number of needle shaped portions 31 of the transplantation device 30 may be the same as the number of pretreatment recesses 21 of the pretreatment tray 20 or smaller than the number of pretreatment recesses 21. The pretreatment interval W2, which is the interval between the adjacent needle shaped portions 31, and the distribution of the needle shaped portions 31, are the interval and distribution of the cell groups Cg, respectively, in the living body T. The interval between the adjacent needle shaped portions 31 and the distribution of the needle shaped portions 31 are appropriately selected so that the cell groups Cg are likely to be active in the living body T.

The needle shaped portions 31 hold the protective liquid Pl to prevent the cells from drying while the cell group Cg to be transplanted is transported to the living body T to thereby prevent the state of the cells from varying in the living body T. When the transplant site is an intradermal or subcutaneous site, the length L3 of the needle shaped portion 31 may be, for example, preferably 200 µm or more and 6 mm or less. The length L3 of the needle shaped portion 31 of 200 µm or more enables stable intradermal transplantation of the graft. The length L3 of the needle shaped portion 31 of 6 mm or less enables transplantation of the cell group Cg into the skin.

The transplantation device 30 includes an engaging portion 32 connected to the proximal end of the needle shaped portion 31. The engaging portion 32 may have a size or a shape that cannot be inserted into the pretreatment recess 21 and engages with part of the pretreatment tray 20 to block insertion of the needle shaped portion 31 in the pretreatment recess 21. That is, the engaging portion 32 stops insertion of the needle shaped portion 31 in the pretreatment recess 21. For example, the engaging portion 32 blocks insertion of the needle shaped portion 31 at a position where the tip of the needle shaped portion 31 does not come into contact with the cell group Cg. Preferably, a plurality of needle shaped portions 31 are collectively supported by the engaging portion 32. As the plurality of needle shaped portions 31 are supported by a single engaging portion 32, variations in the degree of insertion and thus the degree of loading of the graft among the needle shaped portions 31 are suppressed by a single common engaging portion 32.

In transplantation of the cell group Cg that contributes to hair growth or hair restoration, the arrangement of the cell group Cg is determined according to the arrangement of the plurality of needle shaped portions 31. That is, the arrangement of hairs growing from the cell groups Cg is determined. The density of the needle shaped portions 31 is appropriately selected according to the nature of the cell groups Cg to be transplanted. For example, in transplantation of aggregates of cells such as hair follicle germs that contribute to hair growth or hair restoration, the density of the needle shaped portions 31 per unit area is preferably 1/cm² or more and 400/cm² or less, and more preferably 20/cm² or more and 100/cm² or less. When the density of the needle shaped portions 31 is no less than the above lower limit, the density of hairs that grow from the hair follicle germs will provide sufficient hair density. When the density of the needle shaped portions 31 is no more than the above upper limit, nutrients are easily distributed to the cell groups Cg that are positioned in the skin at the same density as that of the needle shaped portions 31. Thus, formation of hair follicles and hairs favorably proceeds.

The transplantation device 30 may include a system for electrically or mechanically detecting whether the cell group Cg is present in the needle shaped portion 31. In loading of the graft into the needle shaped portion 31, the transplantation device 30 including the system for detecting whether the cell group Cg is present prevents the cell group Cg from being left in the pretreatment recess 21 without being loaded into the needle shaped portion 31.

An outer diameter R3 of the needle shaped portion 31 is sufficiently smaller than the inner diameter R2 of the pretreatment recess 21, and is appropriately selected according to the size of the cell group Cg to be transplanted. The outer diameter R3 of the needle shaped portion 31 may be designed, for example, to aspirate a single cell group Cg and not to aspirate two or more cell groups Cg. When the outer diameter R3 of the needle shaped portion 31 is designed to aspirate a single cell group Cg, it is preferred to prevent a pressure loss in the needle shaped portion 31 from becoming excessive during aspiration of the cell group Cg. Preventing a pressure loss in the needle shaped portion 31 from becoming excessive improves the accuracy in aspiration of the cell group Cg through the tip opening of the needle shaped portion 31.

The outer diameter R3 of the needle shaped portion 31 may be substantially equal to the inner diameter R2 of the pretreatment recess 21. When the outer diameter R3 of the needle shaped portion 31 and the inner diameter R2 of the pretreatment recess 21 are substantially equal to each other, the contact area between the outer peripheral surface of the needle shaped portion 31 and the inner peripheral surface of the pretreatment recess 21 increases, reducing a gap formed between the pretreatment recess 21 and the needle shaped portion 31. Accordingly, it is possible to increase a force for aspirating the graft from the pretreatment recess 21 into the needle shaped portion 31. Therefore, the cell groups Cg can be easily loaded into the needle shaped portion 31.

### [Cell Transplantation Pretreatment Method]

A cell transplantation pretreatment method includes a step of transporting the cell group Cg cultured in the culture tray 10 to the pretreatment recess 21 of the pretreatment tray 20, and a step of loading a graft containing the cell group Cg from the pretreatment recess 21 into the transplantation device 30 that performs transplantation into a living body.

The transport step is not limited to transporting the cell group Cg using the transport unit CT. The cell group Cg may also be transported together with the protective liquid Pl contained in the graft, or the cell group Cg may be mechanically held and transported. In the transport step, the protective liquid Pl may be introduced into the pretreatment recess 21 after only the cell group Cg is transported to the pretreatment recess 21 by the transport unit CT, or the cell group Cg may be transported from the culture tray 10 to the pretreatment recess 21 filled with the protective liquid Pl in advance. Holding the cell group Cg together with the protective liquid Pl in the pretreatment recess 21 prevents the cell group Cg from drying, and prevents a change in component ratio of the protective liquid PI. Accordingly, it is possible to hold the cell groups Cg in the pretreatment recesses 21 while maintaining the activity of the cell groups Cg, and thus improve the accuracy in engraftment of the cell groups Cg.

Preferably, the transport step does not affect the state of the cell group Cg. Affecting the state of the cell group Cg includes changes in the shape of the cell group Cg, cell death, directed differentiation, dormancy, cytokine production, expression of specific genes, and the like. The transport step may be performed multiple times to a single culture recess 11 or multiple times to a single pretreatment recess 21, or may be performed from a plurality of culture trays 10 to a plurality of pretreatment trays 20. Thus, the graft can be appropriately held in the culture tray 10 or the pretreatment tray 20 having a shape or color suitable for the purpose to improve the culture, inspection and loading efficiency.

The loading step is a step of loading a graft from the pretreatment recess 21 into the needle shaped portion 31. In the loading step, the needle shaped portion 31 aspirates a graft from the pretreatment recess 21 for loading the graft. That is, a graft is loaded through the tip opening of the needle shaped portion 31, and the cell group Cg is held in the needle shaped portion 31 at a position close to the opening. Accordingly, it is possible to reduce contact between the cell group Cg and the inner peripheral surface of the needle shaped portion 31. Further, since the inner peripheral surface of the needle shaped portion 31 is smooth, the contact area between the cell group Cg and the inner peripheral surface can be minimized. Accordingly, it is possible to prevent the loading step from affecting the cell group Cg, such as causing deformation of the cells.

The cell transplantation pretreatment method may include an inspection step for determining whether the state of cells is suitable for cell transplantation, and making the state of cells in each pretreatment recess 21 suitable for cell transplantation based on the inspection result. Thus, the state of cells suitable for cell transplantation can be achieved in all the needle shaped portions 31.

The inspection step may be, for example, an inspection using a microscope, an optical inspection, or the like for excluding cells that are not suitable for the cell transplantation or checking the state of cells for the cell transplantation. Different inspections may be performed in the culture tray 10 and the pretreatment tray 20. Further, the culture tray 10 and the pretreatment tray 20 may each have a color and a shape suitable for the inspection performed in each of them. For example, cell screening using a microscope may be performed in the culture tray 10, and an optical inspection may be performed in the pretreatment tray 20. In this case, the culture tray 10 may be transparent to improve the visibility under a microscope. Further, the pretreatment tray 20 may be black or white depending on the irradiation light used for the optical inspection.

Alternatively, the culture tray 10 may be black or white, and the pretreatment tray 20 may be transparent. When the pretreatment tray 20 is transparent, the transport step and the loading step can be performed while observing the inside of the pretreatment recesses 21. Accordingly, positioning of the cell groups Cg in the pretreatment recesses 21 and accommodation of the cell groups Cg into the needle shaped portions 31 can be performed with accuracy. In addition, whether the loading step has been appropriately performed can be determined by checking whether no residue remains in the pretreatment recess 21 after the loading step. The transparent pretreatment tray 20 may be made of, for example, a resin such as acrylic, polycarbonate or cycloolefin polymer, or glass.

Further, when an optical inspection is performed, the culture recess 11 or the pretreatment recess 21 preferably has a thin small structure provided with a small shielding portion that prevents light from being guided.

The cell transplantation pretreatment method includes a step of holding a graft in the pretreatment tray 20 during loading of the graft from the culture tray 10 into the transplantation device 30. As the graft is loaded from the culture recess 11 into the pretreatment recess 21, the interface is reconstructed. Due to the reconstruction of the interface, air bubbles can be prevented from entering the pretreatment tray 20. Accordingly, the visibility of the cell group Cg is improved, which improves the efficiency of inspection in the pretreatment recess 21.

The transplantation device 30 in which the graft is loaded in the needle shaped portion 31 injects the graft into a living body. As the tip opening of the needle shaped portion 31 of the transplantation device 30 is inserted through a surface of the living body, the needle shaped portion 31 enters the living body. Then, the graft is released into the living body through the tip opening of the needle shaped portion 31 in a state in which the needle shaped portion 31 is located in the living body. As the graft is released into the living body through the tip opening of the needle shaped portion 31, the graft is positioned in the target site TC at the transplant site, and the cell transplantation is completed. After the transplantation is completed, the needle shaped portion 31 of the transplantation device 30 is removed from the living body.

According to the above embodiment, the following effects can be achieved.
(1) Since the pretreatment recesses 21 and the needle shaped portions 31 are arranged according to the second arrangement, the plurality of needle shaped portions 31 can be simultaneously inserted into the plurality of pretreatment recesses 21, and the grafts can be simultaneously loaded into the plurality of needle shaped portions 31. Therefore, the efficiency in loading the grafts into the transplantation device 30 can be improved.
(2) Since the inner diameter R2 of the pretreatment recess 21 is smaller than the inner diameter of the culture recess 11, a gap formed between the pretreatment recess 21 and the needle shaped portion 31 can be reduced, which increases a force for aspirating the graft into the needle shaped portion 31. Therefore, the accuracy and efficiency in aspiration of the cell groups Cg into the needle shaped portions 31 can be improved.
(3) The arrangement elements in the second arrangement in which the pretreatment recesses 21 are arranged are concentrated in the center part of the pretreatment tray 20 compared with the first arrangement in which the culture recesses 11 are arranged. This prevents the state of the cell groups Cg from varying when the grafts are held in the pretreatment recesses 21. Accordingly, it is possible to prevent the state of the cell groups Cg in the needle shaped portions 31 from varying at the time of loading.
(4) Since the engaging portion 32 is provided to block movement of the needle shaped portion 31 while forming a gap between the tip of the needle shaped portion 31 inserted in the pretreatment recess 21 and the bottom of the pretreatment recess 21, the cell groups Cg can be loaded into the transplantation device while maintaining the state of the needle shaped portions 31 suitable for transplantation during loading of the grafts into the transplantation device.
(5) The pretreatment tray 20 includes a grip portion 23 provided in the outer peripheral portion of the pretreatment tray 20 in which no pretreatment recesses 21 are disposed so that the user can hold the pretreatment tray 20. The pretreatment tray 20 is transferred to a place where transplantation is performed while holding the cell groups Cg from the culture tray 10 in a culture chamber. Since the pretreatment tray 20 having the grip portion 23 can be held by hand, the efficiency in transport of the pretreatment tray 20 can be improved. Therefore, prolongation of the time required for pretreatment of transplantation can be prevented.
(6) In a configuration in which a difference between the first arrangement and the second arrangement satisfies at least one of the above conditions 1 to 3, loading the grafts from the culture recesses into the needle shaped portions requires the needle shaped portions 31 arranged according to the second arrangement to be collectively moved relative to each of the culture recesses 11 to load the grafts one by one into the needle shaped portions 31. In this regard, according to the method in which the pretreatment recesses 21 and the needle shaped portions 31 are arranged according to a common second arrangement, the effects according to the above (1) to (5) are more reliably achieved.
(7) When the depth of the culture recess 11 and the depth of the pretreatment recess 21 are individually set, the depth of the culture recess 11 can be suitably sized for culture and the depth of the pretreatment recess 21 can be suitably sized for loading the graft into the needle shaped portion 31.
(8) When the depth of the pretreatment recess 21 is greater than the depth of the culture recess 11, the grafts can be transported from a plurality of culture recesses 11 to a single pretreatment recess 21 to increase the quantity of grafts to be loaded into the needle shaped portion. Further, when the depth of the pretreatment recess 21 is smaller than the depth of the culture recess 11, the quantity of grafts can be adjusted to the quantity that can be loaded into the needle shaped portion 31.

The above embodiment can be modified and implemented as follows.

### [Arrangement]

As shown in Fig. 4, the pretreatment recesses 21 and the needle shaped portions 31 may be arranged in an annular shape, while the culture recesses 11 are arranged in a matrix. In this case, the pretreatment recesses 21 and the needle shaped portions 31 may be arranged to satisfy all the above conditions 1 to 3. That is, the culture interval W1 and the pretreatment interval W2 may be different from each other, and the number of culture recesses 11 in the first arrangement direction D1 and the number of pretreatment recesses 21 in the second arrangement direction D2 may be different from each other.

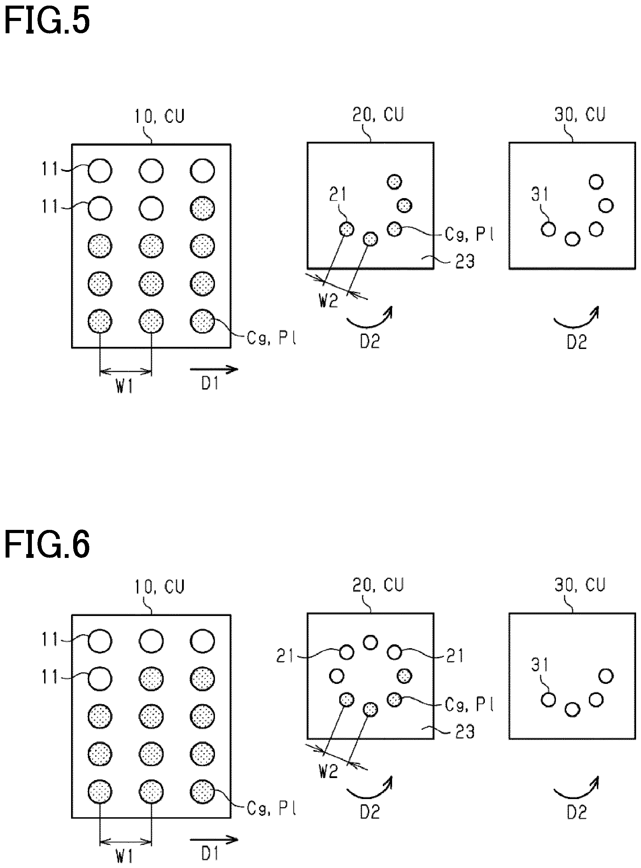

Further, as shown in Fig. 5, the number of pretreatment recesses 21 and the number of needle shaped portions 31 in the second arrangement direction D2 may be different from the number of culture recesses 11 in the first arrangement direction D1. That is, the pretreatment recesses 21 and the needle shaped portions 31 may be arranged to satisfy the above conditions 1 and 2. For example, five pretreatment recesses 21 and five needle shaped portions 31 may be arranged in the second arrangement direction D2, while three culture recesses 11 are arranged in the first arrangement direction D1.

Further, as shown in Fig. 6, the number of needle shaped portions 31 in the second arrangement direction D2 may be different from the number of pretreatment recesses 21 in the second arrangement direction D2. For example, four needle shaped portions 31 may be arranged in the second arrangement direction D2, and the second arrangement may be composed of four arrangement elements arranged in the second arrangement direction D2.

The pretreatment recesses 21 and the needle shaped portions 31 may be arranged in a concentric annular shape, an elliptical annular shape, a polygonal ring shape, a concentric polygonal ring shape, a radial shape, or an irregular shape.

### [Bottom Shape]

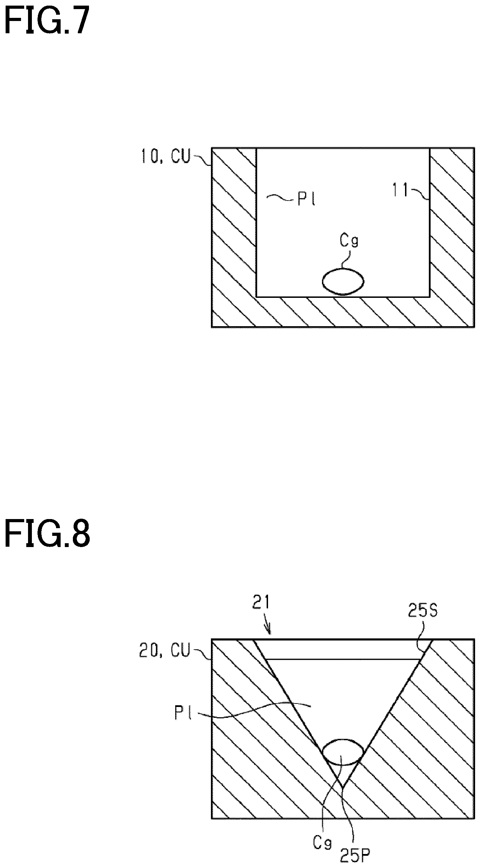

A bottom shape of the culture recess 11 and a bottom shape of the pretreatment recess 21 may be different from each other. As shown in Fig. 7, the culture recess 11 has a bottom shape suitable for culturing the cell group Cg, for example, a flat shape. For example, when the cell group Cg is an adhesive cell, the cell group Cg can be uniformly adhered to the bottom of the culture recess 11, which facilitates cell proliferation.

As shown in Fig. 3, the pretreatment recess 21 has a bottom shape suitable for loading the graft into the needle shaped portion 31, for example, a hemispherical recess. When the graft is aspirated from the pretreatment recess 21 into the needle shaped portion 31, the cell group Cg is centered on the bottom since the pretreatment recess 21 has a hemispherical bottom. Therefore, the cell group Cg can be easily loaded into the needle shaped portion 31. The pretreatment recess 21 may have a shape in which the cell group Cg is centered, and may have, for example, a spindle shape or a V-shaped cross-section in which the apex is located at the center of the bottom.

Fig. 8 illustrates an example of the pretreatment recess 21 having a V-shaped cross-section. As shown in Fig. 8, the pretreatment recess 21 may have an apex 25P located on the bottom and an inner peripheral surface 25S tapered toward the apex 25P. For example, the pretreatment recess 21 may have a shape of a pyramidal or conical recess. When the pretreatment recess 21 has such a shape, the cell group Cg settled in the pretreatment recess 21 is held at a position away from the bottom. Therefore, compared with the case where the bottom is a flat or curved surface and the cell group Cg has a surface contact with the bottom of the pretreatment recess 21, the contact area between the pretreatment recess 21 and the cell group Cg can be reduced. Accordingly, the load applied on the cell group Cg is reduced.

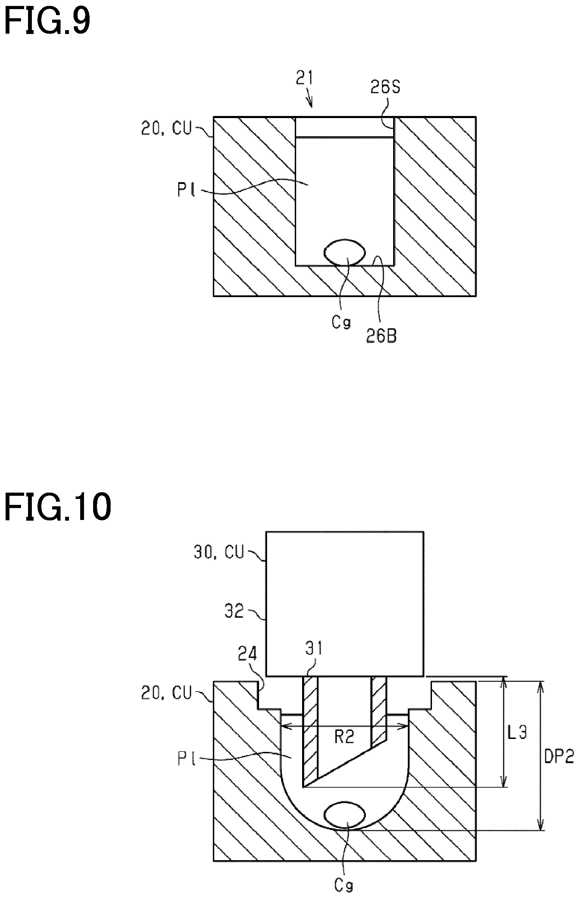

As shown in Fig. 9, the pretreatment recess 21 may have a shape with a flat bottom 26B and an inner peripheral surface 26S vertically extending from the bottom 26B. For example, the pretreatment recess 21 may have the shape of a cylindrical or prismatic recess. When the pretreatment recess 21 has such a shape, the cell group Cg is prevented from adhering to the inner peripheral surface 26S, and the cell group Cg when settled in the pretreatment recess 21 is located on the bottom 26B, that is, at the deepest position in the pretreatment recess 21. Since the position of the cell group Cg in the pretreatment recess 21 in the depth direction is specified, the positional relationship between the needle shaped portion 31 and the cell group Cg can be more accurately defined in accommodation of the cell group Cg into the needle shaped portion 31. Therefore, the cell group Cg can be aspirated with an appropriate force. As a result, even when the cell group Cg has a low cohesive force and tends to easily decompose, it is possible to collect the cell group Cg into the needle shaped portion 31 with accuracy.

### [Engaging Portion]

As shown in Fig. 10, the pretreatment recess 21 may further include an engaged portion 24 configured to engage with the engaging portion 32. The engaged portion 24 may be, for example, a recess formed by enlarging the opening of the pretreatment recess 21, and configured to block or stop lowering of the engaging portion 32. Accordingly, the transplantation device 30 can be positioned so that the tip of the needle shaped portion 31 does not come into contact with the cell group Cg. When the engaging portion 32 engages with the engaged portion 24, a gap is formed between the tip of the needle shaped portion 31 and the bottom of the pretreatment recess 21. The transplantation device 30 positioned so as not to come into contact with the cell group Cg can perform loading of the cell group Cg into the needle shaped portion 31 while maintaining the state of the cell group Cg as it is before the loading.

A plurality of engaged portions 24 may be formed in a single pretreatment recess 21, or the engaged portions 24 may have a multi-stage structure. When the pretreatment recess 21 has a multi-stage engaged portion 24, for example, two types of transplantation devices 30 having an engaging portion 32 having different sizes can individually engage with the pretreatment recess 21. Further, the pretreatment recess 21 having the multi-stage engaged portion 24 can engage with, for example, the transplantation device 30 having a multi-stage engaging portion 32. Engagement between the multi-stage engaged portion 24 and the multi-stage engaging portion 32 can enhance the engagement force between the pretreatment tray 20 and the transplantation device 30 compared with engagement between a single-stage engaged portion 24 and a single-stage engaging portion 32.

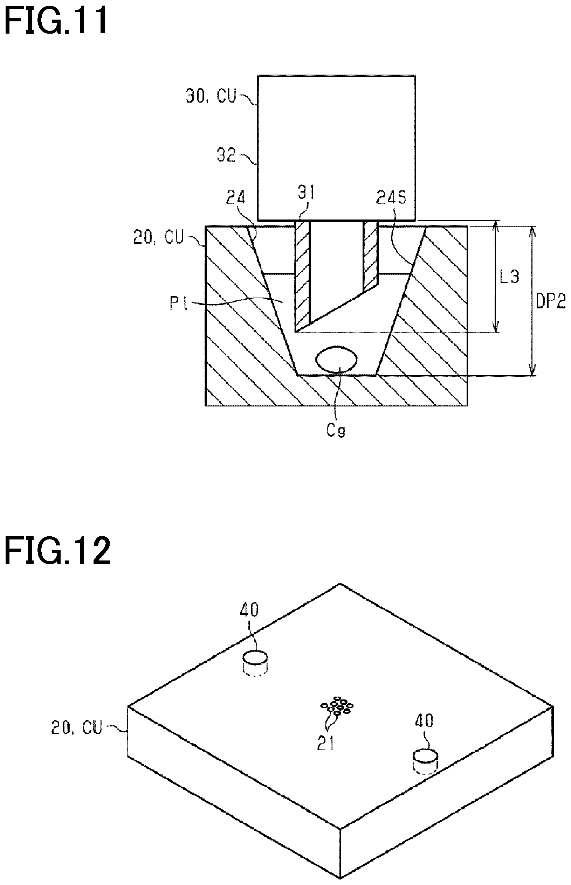

As shown in Fig. 11, the pretreatment recess 21 may have an inner peripheral surface 24S configured to function as an engaged portion. For example, when the inner peripheral surface 24S of the pretreatment recess 21 has a tubular frustum shape tapered toward the bottom, the inner peripheral surface of the pretreatment recess 21 and the engaging portion 32 engage to block lowering of the needle shaped portion 31.

### [Positioning Structure]

The pretreatment tray 20 and the transplantation device 30 may have a structure for positioning the transplantation device 30 relative to the pretreatment tray 20 in a direction perpendicular to the depth direction of the pretreatment recess 21.

The positioning structure formed in the pretreatment tray 20 may be a three-dimensional structure which is recessed or protruding from the upper surface of the pretreatment tray 20 in the depth direction of the pretreatment recess 21. The positioning structure formed in the transplantation device 30 may be a three-dimensional structure that fits with the three-dimensional structure of the pretreatment tray 20.

For example, the positioning structure formed in the pretreatment tray 20 may be a hole or a protrusion located on the outer peripheral portion of the pretreatment tray 20. Fig. 12 illustrates an example of a reference structure 40 as the positioning structure. Fig. 12 illustrates an example in which the reference structure 40 is a hole. When the reference structure 40 is a hole, the transplantation device 30 has a protrusion that fits with the hole. When the reference structure 40 is a protrusion, the transplantation device 30 has a hole that fits with the protrusion. The corresponding structure formed in the transplantation device 30, such as a protrusion or a hole, may be located, for example, on the distal end surface of the engaging portion 32 or the outer periphery of the engaging portion 32.

The pretreatment tray 20 includes two or more reference structures 40. Since engagement between the pretreatment tray 20 and the transplantation device 30 is made at two or more positions, the transplantation device 30 can be accurately positioned relative to the pretreatment tray 20. The positions of the reference structures 40 are not limited as long as the reference structures 40 are provided in a region of the pretreatment tray 20 surrounding a region in which a plurality of pretreatment recesses 21 are formed. For example, two reference structures 40 may be provided at positions on opposed sides of the region in which the plurality of pretreatment recesses 21 are formed.

In at least one of the plurality of reference structures 40, a size of the reference structure 40 in a direction perpendicular to the depth direction may be slightly larger than that of a corresponding structure of the transplantation device 30 corresponding to the reference structure 40. A difference in size between the corresponding structure and the reference structure 40 is set so that the respective needle shaped portions 31 are positioned at positions where they can be inserted into the respective pretreatment recesses 21 even when the corresponding structure is displaced from the reference structure 40. Since the reference structure 40 is larger in size than the corresponding structure, the reference structure 40 and the corresponding structure can be easily engaged with each other. This is particularly effective when positioning of the transplantation device 30 relative to the pretreatment tray 20 is manually performed.

For example, when the corresponding structure is a cylindrical protrusion extending in the depth direction, the reference structure 40 may be a cylindrical hole having a diameter larger than a diameter of the corresponding structure. Further, the reference structure 40 may also be an elliptical cylindrical hole having a minor diameter larger than or equal to a diameter of the corresponding structure. When the reference structure 40 has an elliptical cylindrical shape, a gap is likely to be formed between the reference structure 40 and the corresponding structure. Accordingly, the reference structure 40 and the corresponding structure can be easily engaged with each other.

In addition, two reference structures 40 may include, for example, a first reference structure 40 and a second reference structure 40. The first reference structure 40 may have the same diameter as that of a first corresponding structure and the second reference structure 40 may have a diameter larger than that of a second corresponding structure. The diameters of the two corresponding structures are the same, and the diameters of the two reference structures 40 are different from each other. With this configuration, the first reference structure 40 and the first corresponding structure are engaged with each other, and then the second reference structure 40 and the second corresponding structure are engaged with each other. As a result, even when the movement of the transplantation device 30 is restricted by the engagement between the first reference structure 40 and the first corresponding structure, there is a margin for the position of the second corresponding structure engageable with the second reference structure 40, and the second reference structure 40 and the second corresponding structure can be easily engaged with each other. Therefore, positioning of the transplantation device 30 relative to the pretreatment tray 20 can be easily performed with accuracy.

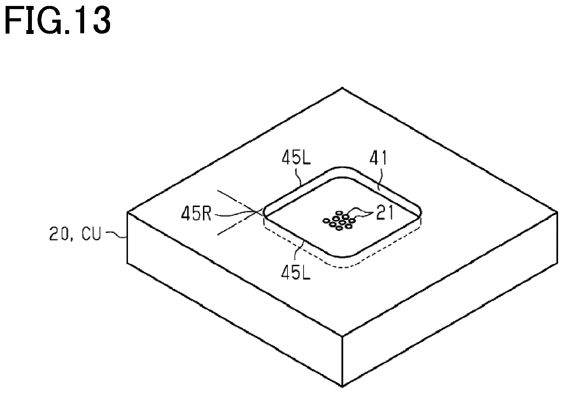

Further, the positioning structure formed in the pretreatment tray 20 may be a structure having a linear portion extending linearly when viewed in a direction parallel to the depth direction. Fig. 13 illustrates an example of a reference structure 41 as the positioning structure. The reference structure 41 is a substantially rectangular frame-shaped step surrounding the plurality of pretreatment recesses 21. The reference structure 41 includes linear portions 45L at positions corresponding to two sides meeting at a corner of the rectangle.

The two linear portions 45L are located on two straight lines intersecting each other, and the intersection of these straight lines is a reference point 45R. When the reference structure 41 includes at least two linear portions 45L, the position of the needle shaped portion 31 relative to the reference point 45R is accurately determined by aligning the transplantation device 30 with the linear portions 45L. Accordingly, the accuracy of alignment between the pretreatment recesses 21 and the needle shaped portions 31 is improved. A portion of the transplantation device 30 that can be aligned with the linear portions 45L may be, for example, a side face of the engaging portion 32.

The linear portions 45L may alternatively correspond to two sides of a polygon, not only a rectangle. When the reference structure 41 includes, in addition to the two linear portions 45L, a portion corresponding to other side of a polygon, a gap may be formed between the portion corresponding to other side and the transplantation device 30. In the above configuration in which a gap is formed between a portion of the reference structure 41 other than the two linear portions 45L and the transplantation device 30, the transplantation device 30 can be easily engaged with the reference structure 41. Accordingly, positioning of the transplantation device 30 relative to the pretreatment tray 20 can be easily performed.

As shown in Fig. 13, when the pretreatment recesses 21 are located on a bottom of a recess formed by the reference structure 41, the reference structure 41 may be formed by, for example, counter-boring. The reference structure 41 is not limited to the above structure, and may also be a structure protruding from the upper surface of the pretreatment tray 20 with the pretreatment recesses 21 formed on the top, or a ridge structure surrounding the pretreatment recesses 21>

The two linear portions 45L may be connected to each other at their ends, or may not be necessarily connected to each other as long as the extension lines of the linear portions 45L intersect each other.

### [Needle Shaped Portion]

The length L3 of the needle shaped portions 31 adj acent to each other may vary according to the shape of the surface of the living body. In this case, if the second arrangement is a symmetrical arrangement, the needle shaped portion 31 having a large length L3 may be inserted into the pretreatment recess 21 having a small depth DP2. Therefore, it is preferred that at least one of the pretreatment tray 20 and the transplantation device 30 includes a positioning structure for matching the depth DP2 of the pretreatment recess 21 with the length L3 of the needle shaped portion 31. As the length of the needle shaped portions 31 are designed according to the unevenness of the surface of the living body, the grafts can be transplanted at a constant distance from the surface of the living body. Accordingly, the cell groups Cg can be transplanted at an appropriate depth in the living body, and the activity of the transplanted cell groups Cg can be enhanced.

### [Supplementary Notes]

The above means for solving the problems embrace the following technical ideas derived from the above embodiment and modifications thereof.

A pretreatment tray for use in combination with each of a culture tray including a plurality of culture recesses arranged according to a first arrangement and a transplantation device including a plurality of needle shaped portions arranged according to a second arrangement, the needle shaped portions each having a tubular shape configured to allow entry and exit of a graft containing the cell group, wherein
the pretreatment tray includes a plurality of pretreatment recesses arranged according to the second arrangement, and is configured such that cell groups cultured in the plurality of culture recesses are transported to the plurality of pretreatment recesses and the cell groups are loaded simultaneously from the plurality of pretreatment recesses into the plurality of needle shaped portions.

### [Reference Signs List]

- 10: Culture tray
- 11: Culture recess
- 20: Pretreatment tray
- 21: Pretreatment recess
- 23: Grip portion
- 24: Engaged portion
- 30: Transplantation device
- 31: Needle shaped portion
- 32: Engaging portion

## Claims

1. A cell transplantation pretreatment method comprising the steps of:
transporting a cell group cultured in a culture recess to a pretreatment recess using a culture tray including a plurality of the culture recesses arranged according to a first arrangement, a pretreatment tray including a plurality of the pretreatment recesses arranged according to a second arrangement, and a transplantation device including a needle shaped portion having a tubular shape configured to allow entry and exit of a graft containing the cell group, the transplantation device including a plurality of the needle shaped portions arranged according to the second arrangement; and
loading the cell groups simultaneously from the plurality of pretreatment recesses into the plurality of needle shaped portions.

2. The cell transplantation pretreatment method according to claim 1, wherein the pretreatment recess has an inner diameter smaller than that of the culture recess.

3. The cell transplantation pretreatment method according to claim 1 or 2, wherein
an engaging portion is provided to block insertion of the needle shaped portion while forming a gap between a tip of the needle shaped portion inserted in the pretreatment recess and a bottom of the pretreatment recess, and
the step of loading the cell groups into the needle shaped portions is performed in a state in which the engaging portion blocks insertion of the needle shaped portion.

4. The cell transplantation pretreatment method according to any one of claims 1 to 3, wherein the pretreatment recess has a bottom shape different from that of the culture recess.

5. The cell transplantation pretreatment method according to any one of claims 1 to 4, wherein
the first arrangement and the second arrangement satisfy at least one of the following conditions 1 to 3:
(Condition 1) an arrangement direction of the culture recesses arranged according to the first arrangement and an arrangement direction of the pretreatment recesses arranged according to the second arrangement are different from each other;
(Condition 2) an interval between the culture recesses adjacent to each other and an interval between the pretreatment recesses adjacent to each other are different from each other; and
(Condition 3) a number of culture recesses in the arrangement direction of the culture recesses arranged according to the first arrangement and a number of pretreatment recesses in the arrangement direction of the pretreatment recesses arranged according to the second arrangement are different from each other.

6. The cell transplantation pretreatment method according to any one of claims 1 to 5, wherein the culture recess has a depth different from that of the pretreatment recess.

7. The cell transplantation pretreatment method according to any one of claims 1 to 6, wherein
the pretreatment recess has a shape in which an outer peripheral surface of the needle shaped portion is able to fit, and
the step of loading the cell groups into the needle shaped portions is performed in a state in which the outer peripheral surface of the needle shaped portion is fitted in the pretreatment recess.

8. The cell transplantation pretreatment method according to any one of claims 1 to 7, wherein the pretreatment tray and the transplantation device include a structure for positioning the transplantation device relative to the pretreatment tray in a direction perpendicular to a depth direction of the pretreatment recess.

9. The cell transplantation pretreatment method according to any one of claims 1 to 8, wherein the pretreatment tray is transparent.

10. A cell transplantation pretreatment device comprising:
a transport unit that transports a cell group cultured in a culture recess to a pretreatment recess using a culture tray including a plurality of the culture recesses arranged according to a first arrangement and a pretreatment tray including a plurality of the pretreatment recesses arranged according to a second arrangement; and
a loading unit that loads the cell groups simultaneously from the plurality of pretreatment recesses into a plurality of needle shaped portions using a transplantation device and the pretreatment tray, the transplantation device including the plurality of needle shaped portions having a tubular shape configured to allow entry and exit of a graft containing the cell group, the plurality of needle shaped portions being arranged according to the second arrangement.

11. A cell transplantation pretreatment unit comprising:
a culture tray including a plurality of culture recesses arranged according to a first arrangement;
a pretreatment tray including a plurality of pretreatment recesses arranged according to a second arrangement, the pretreatment tray being configured such that cell groups cultured in the plurality of culture recesses are transported to the plurality of pretreatment recesses; and
a transplantation device including a plurality of needle shaped portions having a tubular shape configured to allow entry and exit of a graft containing the cell group, the plurality of needle shaped portions being arranged according to the second arrangement, and the transplantation device being configured such that the cell groups are able to be loaded simultaneously from the plurality of pretreatment recesses into the plurality of needle shaped portions.
